# EUROPÄISCHE PATENTANMELDUNG

(11) **EP 3 070 160 A1**
(43) Veröffentlichungstag der Anmeldung: **21.09.2016**
(21) Anmeldenummer: 16159906.3
(22) Anmeldetag: 11.03.2016
(51) Int. Cl.: C12M 3/06, C12M 1/12, C12M 1/42, C12M 1/00

(54) **VORRICHTUNG UND VERFAHREN ZUM BEARBEITEN EINER ZIELZELLEN ENTHALTENDEN PROBE BIOLOGISCHEN MATERIALS**

(30) Priorität: 17.03.2015 DE 102015204793
(71) Anmelder: ROBERT BOSCH GMBH, 70442 Stuttgart (DE)
(72) Erfinder: Mueller-Fiedler, Roland, 71229 Leonberg (DE); Brettschneider, Thomas, 71229 Leonberg (DE); Hoffmann, Jochen, 71229 Leonberg (DE)

(57) **Zusammenfassung**

Die Erfindung betrifft eine Vorrichtung (100) zum Bearbeiten einer Zielzellen enthaltenden Probe biologischen Materials. Die Vorrichtung (100) weist eine Membran (107) mit Poren (108) auf. Hierbei sind die Poren (108) ausgebildet, um die Zielzellen aus der Probe zu filtern. Die Membran (107) ist aus einem piezoelektrischen Material ausgeformt. Dabei ist die Membran (107) durch ein elektrisches Signal mechanisch verformbar, um eine Lyse der Zielzellen zu bewirken.

## Beschreibung

### Stand der Technik

Die vorliegende Erfindung bezieht sich auf eine Vorrichtung zum Bearbeiten einer Zielzellen enthaltenden Probe biologischen Materials, auf ein Verfahren zum Herstellen einer Vorrichtung zum Bearbeiten einer Zielzellen enthaltenden Probe biologischen Materials, auf ein Verfahren zum Bearbeiten einer Zielzellen enthaltenden Probe biologischen Materials, auf ein entsprechendes Steuergerät sowie auf ein entsprechendes Computerprogramm.

Mikrofluidische Lab-on-a-Chip-Systeme können häufig beispielsweise aus Mehrschichtaufbauten mit integrierten mikrofluidischen Kanälen, Kavitäten, Ventilen und Filtereinrichtungen bestehen.

Die US 6,100,084 offenbart eine Mikro-Ultraschallbehandlungseinrichtung zur Lyse von Sporen. Hierbei wird beispielsweise eine Lyse durch einen in ein mikrofluidisches System integrierten Ultraschallgeber durchgeführt, wobei eine Filtrierung in einem separaten Teil des Systems durchgeführt wird und das Filtrat anschließend in eine Lysekammer überführt wird.

### Offenbarung der Erfindung

Vor diesem Hintergrund werden mit dem hier vorgestellten Ansatz eine Vorrichtung zum Bearbeiten einer Zielzellen enthaltenden Probe biologischen Materials, ein Verfahren zum Herstellen einer Vorrichtung zum Bearbeiten einer Zielzellen enthaltenden Probe biologischen Materials, ein Verfahren zum Bearbeiten einer Zielzellen enthaltenden Probe biologischen Materials, weiterhin ein Steuergerät, das eines dieser Verfahren verwendet, sowie schließlich ein entsprechendes Computerprogramm gemäß den Hauptansprüchen vorgestellt. Vorteilhafte Ausgestaltungen ergeben sich aus den jeweiligen Unteransprüchen und der nachfolgenden Beschreibung.

Gemäß Ausführungsformen der vorliegenden Erfindung kann insbesondere für mikrofluidische Chiplabor-Systeme bzw. Lab-on-Chip-Systeme zur medizinischen Diagnostik eine funktionale Vereinigung von Filtration und Lyse in einem einzigen mikrofluidischen Bauteil realisiert werden. Somit kann beispielsweise ein einziges Bauteil als kombinierter mikrofluidischer piezoelektromechanischer Filter und Lysator für ein mikrofluidisches Lab-on-Chip-System bereitgestellt und verwendet werden.

Vorteilhafterweise können gemäß Ausführungsformen der vorliegenden Erfindung insbesondere dadurch, dass sowohl Filtration als auch Lyse in einer Kammer und mittels eines Bauteils durchgeführt werden können, hierbei Kosten, Platzbedarf und Komplexität einer mikrofluidischen Verschaltung reduziert werden. Auch kann beispielsweise eine Empfindlichkeit bzw. Sensitivität eines mikrofluidischen Gesamtsystems erhöht werden, da die Probe, insbesondere die Zielzellen, zwischen Filtration und Lyse nicht zwischen zwei Kammern transferiert werden muss und somit kein Probenmaterial auf der Strecke bleibt.

Es kann gemäß Ausführungsformen der vorliegenden Erfindung ferner insbesondere eine Verwendung eines externen Ultraschallgebers vermieden werden, dessen Einkopplung von einem Kontakt des Ultraschallgebers zu einer Oberfläche des mikrofluidischen Systems abhängig wäre, d. h., es kann ein diesbezüglicher Aufwand für eine Justage entfallen. Da somit beispielsweise mikrofluidische Systeme mit mikrosystemtechnischen Methoden in großen Stückzahlen mit geringen Toleranzen hergestellt werden können, so können eine Reproduzierbarkeit und eine Ausbeute bei der Lyse von Zellen erhöht werden, wodurch wiederum eine Sensitivität des Gesamtsystems gesteigert werden kann. Zusätzlich können Kosten für eine ansonsten erforderliche externe Ansteuerungseinheit reduziert werden, da auf einen externen Ultraschallgeber und eine zugehörige Mechanik verzichtet werden kann. Zudem kann eine mechanische Belastung eines mikrofluidischen Systems gesenkt werden, da anders als bei einer Verwendung eines externen Ultraschallgebers, bei dem sich der Ultraschall über die Wände der Lysekammer hinaus ausbreiten kann, eine lokale und gezielte Einbringung von Energie einfach erreicht werden kann. Somit kann eine Ultraschallenergie auf gezielte Weise lediglich dort eingekoppelt werden, wo sie tatsächlich benötigt wird. Hierdurch kann eine mechanische Belastung reduziert und eine Robustheit des Gesamtsystems erhöht werden. Anders als bei enzymatischen Ansätzen zur Lyse kann auf zusätzliche Reagenzien verzichtet werden, wodurch Kosten gespart werden können und eine Komplexität einer mikrofluidischen Verschaltung reduziert werden kann. Im Gegensatz zu thermischen Ansätzen zur Lyse kann auf eine Ankopplung bzw. Einbringung eines Heizelements verzichtet werden, wobei die Lyse auch beispielsweise bei Raumtemperatur durchgeführt werden kann. Dies kann eine Extraktion von Proteinen ermöglichen, welche bei einer thermischen Lyse denaturiert werden würden.

Es wird eine Vorrichtung zum Bearbeiten einer Zielzellen enthaltenden Probe biologischen Materials vorgestellt, wobei die Vorrichtung folgende Merkmale aufweist:
eine Membran mit Poren, wobei die Poren ausgebildet sind, um die Zielzellen aus der Probe zu filtern, wobei die Membran aus einem piezoelektrischen Material ausgeformt ist, wobei die Membran durch ein elektrisches Signal mechanisch verformbar ist, um eine Lyse der Zielzellen zu bewirken.

Bei der Vorrichtung kann es sich um ein mikrofluidisches System oder einen Teil eines mikrofluidischen Systems handeln, insbesondere um ein sogenanntes Chiplabor-System oder Chiplabor, das auch als Lab-on-Chip-System bezeichnet werden kann. Somit kann ein mikrofluidisches System die Vorrichtung aufweisen. Das piezoelektrische Material kann beispielsweise ein piezoelektrisches Keramikmaterial aufweisen, insbesondere Blei-Zirkonat-Titanat (PZT), ein Stoffverbund aus Blei (Pb), Sauerstoff (O) und Titan (Ti) oder Zirconium (Zr). Die Probe kann eine flüssige Probe sein. Die Vorrichtung kann ausgebildet sein, um die Probe an die Membran und zusätzlich oder alternativ durch die Membran zu spülen. Jede Pore der Membran kann eine Abmessung aufweisen, die minimal 10 % der Abmessungen einer einzelnen Zielzelle und einer Abmessung einer Begleitzelle und zusätzlich oder alternativ eines Begleitpartikels der Probe liegt. Die Membran kann ausgebildet sein, um durch das elektrische Signal in mechanische Schwingungen versetzbar zu sein. Bei dem elektrischen Signal kann es sich um ein elektrisches Wechselsignal handeln.

Gemäß einer Ausführungsform kann die Membran zumindest an einer ersten Hauptoberfläche mit einer ersten elektrisch leitfähigen Schicht und an einer zweiten Hauptoberfläche mit einer zweiten elektrisch leitfähigen Schicht beschichtet sein. Eine solche Ausführungsform bietet den Vorteil, dass ein elektrisches Feld im Wesentlichen normal zu einer Hauptoberfläche der Membran angelegt werden kann, wodurch eine mechanische Auslenkung der Membran normal zur Oberfläche begrenzt werden kann. Hierdurch kann eine Belastung an Befestigungspunkten der Membran gesenkt und eine Robustheit der Vorrichtung erhöht werden. Auch kann ein direkter Kontakt zwischen der Probe und einer Oberfläche der Membran vermieden werden und damit eine Freisetzung von Inhibitoren für biochemische Reaktionen verhindert werden.

Auch kann die Vorrichtung elektrische Leiter zum elektrischen Kontaktieren der Membran aufweisen. Hierbei kann ein erster elektrischer Leiter auf Seiten einer ersten Hauptoberfläche der Membran angeordnet sein und kann ein zweiter elektrischer Leiter auf Seiten einer zweiten Hauptoberfläche der Membran angeordnet sein. Die erste Hauptoberfläche und die zweite Hauptoberfläche der Membran können voneinander abgewandt sein. Eine solche Ausführungsform bietet den Vorteil, dass eine Ankontaktierung der Membran zur mechanischen Verformung durch das elektrische Signal auf einfache und unaufwendige Weise realisiert werden können.

Alternativ kann die Vorrichtung elektrische Leiter zum elektrischen Kontaktieren der Membran aufweisen. Hierbei können die elektrischen Leiter auf Seiten einer ersten Hauptoberfläche der Membran angeordnet sein. Dabei kann die Membran einen Durchkontakt zwischen der ersten Hauptoberfläche und einer zweiten Hauptoberfläche der Membran aufweisen, wobei einer der elektrischen Leiter an dem Durchkontakt angeschlossen sein kann. Die erste Hauptoberfläche und die zweite Hauptoberfläche der Membran können voneinander abgewandt sein. Der eine der elektrischen Leiter kann elektrisch leitfähig mit dem Durchkontakt verbunden sein. Eine solche Ausführungsform bietet den Vorteil, dass lediglich auf einer Seite der Membran elektrische Leiter vorgesehen sein brauchen, wobei ein Aufbau der Vorrichtung vereinfacht werden kann.

Ferner kann die Vorrichtung eine Trägerschicht mit einem Aussparungsabschnitt zum Aufnehmen der Membran aufweisen. Hierbei kann die Membran in dem Aussparungsabschnitt der Trägerschicht angeordnet sein. Die Trägerschicht kann aus einem elastischen Material ausgeformt sein. Der Aussparungsabschnitt kann durch einen Vertiefungsabschnitt oder ein Durchgangsloch ausgeformt sein. Eine solche Ausführungsform bietet den Vorteil, dass die Membran vor mechanischer Belastung geschützt sowie sicher in Position gehalten unterbringbar ist. Ferner kann im Falle eines Schichtstapelaufbaus der Vorrichtung die Trägerschicht als eine Zwischenlage mit Schutzfunktion, Dichtfunktion und/oder Positionierfunktion fungieren.

Gemäß einer Ausführungsform kann die Vorrichtung ein erstes Substrat mit einer ersten Kavität, ein zweites Substrat mit einer zweiten Kavität und elektrische Leiter zum elektrischen Kontaktieren der Membran aufweisen. Hierbei können die Substrate zusammengefügt sein. Dabei können die Membran und die elektrischen Leiter zwischen den Substraten angeordnet sein. Hierbei können mittels der Membran die Kavitäten voneinander abgetrennt sein. Somit können die Substrate aneinandergefügt sein. Anders ausgedrückt können die Substrate mittels einer Fügeverbindung miteinander verbunden sein. Dabei können die Substrate aufeinandergestapelt angeordnet und miteinander verbunden sein. Mittels der Membran können die Kavitäten teilweise fluidisch voneinander abgetrennt sein, wobei die Membran für Partikel mit einer Abmessung bis zu einem Durchmesser einer Pore der Membran permeabel sein kann. Eine solche Ausführungsform bietet den Vorteil, dass ein unaufwendiger und stabiler Schichtaufbau bzw. Schichtstapel bereitgestellt werden kann, in dem die Membran anordenbar oder angeordnet ist.

Dabei kann zumindest eines der Substrate einen Aufnahmeabschnitt zum Aufnehmen der Membran aufweisen. Hierbei kann die Membran in dem Aufnahmeabschnitt angeordnet sein. Insbesondere kann eine Fügefläche, an der ein Substrat mit dem anderen Substrat zusammenfügbar ist, um einen Bereich der Kavität herum ein gestuftes Profil und zusätzlich oder alternativ einen Vertiefungsabschnitt aufweisen. Eine solche Ausführungsform bietet den Vorteil, dass die Membran in Zwischenlage zwischen den Substraten optional auch ohne eine Trägerschicht sicher gehalten sein kann.

Es wird auch ein Verfahren zum Herstellen einer Vorrichtung zum Bearbeiten einer Zielzellen enthaltenden Probe biologischen Materials vorgestellt, wobei das Verfahren folgende Schritte aufweist:

Ausformen einer Membran aus einem piezoelektrischen Material, wobei die Membran durch ein elektrisches Signal mechanisch verformbar ist, um eine Lyse der Zielzellen zu bewirken; und

Ausbilden von Poren, die zum Filtern der Zielzellen aus der Probe geeignet sind, in der Membran.

Durch Ausführen des Verfahrens zum Herstellen kann eine Ausführungsform der vorstehend genannten Vorrichtung zum Bearbeiten vorteilhaft hergestellt werden.

Es wird ferner ein Verfahren zum Bearbeiten einer Zielzellen enthaltenden Probe biologischen Materials, wobei das Verfahren in Verbindung mit einer Ausführungsform der vorstehend genannten Vorrichtung ausführbar ist, wobei das Verfahren folgende Schritte aufweist:

Filtern der Zielzellen aus der Probe unter Verwendung der Membran mit den Poren; und

Anlegen eines elektrischen Signals an die aus dem piezoelektrischen Material ausgeformte Membran, um die Membran mechanisch zu verformen, um eine Lyse der Zielzellen zu bewirken.

Das Verfahren zum Bearbeiten kann in Verbindung mit einer Ausführungsform der vorstehend genannten Vorrichtung zum Bearbeiten vorteilhaft ausgeführt werden. Im Schritt des Anlegens kann als das elektrische Signal eine elektrische Wechselspannung an die Membran angelegt werden.

Der hier vorgestellte Ansatz schafft ferner ein Steuergerät, das ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form eines Steuergeräts kann die der Erfindung zugrunde liegende Aufgabe schnell und effizient gelöst werden.

Unter einem Steuergerät kann vorliegend ein elektrisches Gerät verstanden werden, das Sensorsignale verarbeitet und in Abhängigkeit davon Steuer- und/oder Datensignale ausgibt. Das Steuergerät kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen des Steuergeräts beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einem Steuergerät ausgeführt wird.

Der hier vorgestellte Ansatz wird nachstehend anhand der beigefügten Zeichnungen beispielhaft näher erläutert. Es zeigen:
Fig. 1 eine schematische Schnittdarstellung einer Vorrichtung zum Bearbeiten gemäß einem Ausführungsbeispiel der vorliegenden Erfindung;
Fig. 2 eine schematische Schnittdarstellung einer Vorrichtung zum Bearbeiten gemäß einem weiteren Ausführungsbeispiel der vorliegenden Erfindung;
Fig. 3 eine schematische Schnittdarstellung einer Vorrichtung zum Bearbeiten gemäß einem weiteren Ausführungsbeispiel der vorliegenden Erfindung;
Fig. 4 eine schematische Schnittdarstellung einer Vorrichtung zum Bearbeiten gemäß einem weiteren Ausführungsbeispiel der vorliegenden Erfindung;
Fig. 5 ein Ablaufdiagramm eines Verfahrens zum Herstellen gemäß einem Ausführungsbeispiel der vorliegenden Erfindung; und
Fig. 6 ein Ablaufdiagramm eines Verfahrens zum Bearbeiten gemäß einem Ausführungsbeispiel der vorliegenden Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

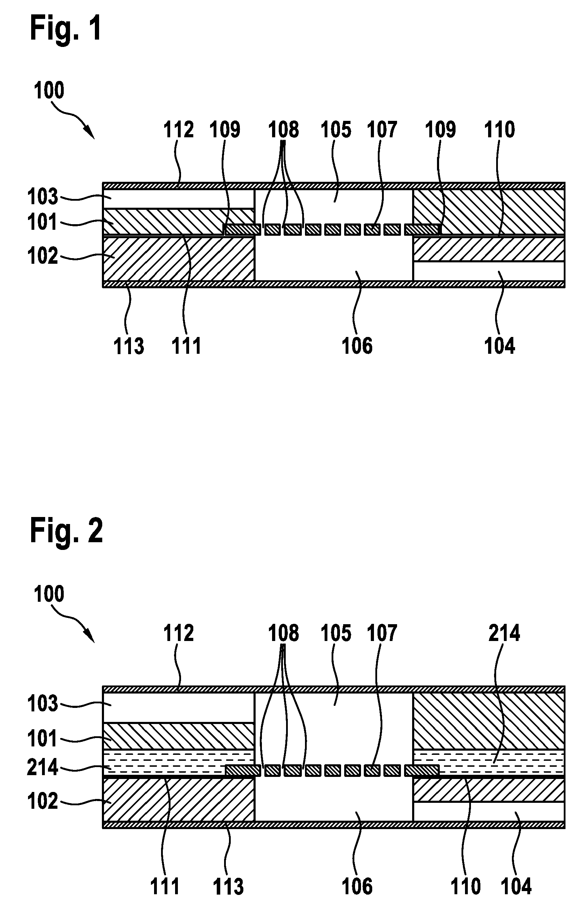

Bevor Ausführungsbeispiele der vorliegenden Erfindung unter Bezugnahme auf die Figuren 1 bis 6 beschrieben werden, wird zunächst eine allgemeine Einführung zu sogenannten Lab-on-a-Chip-Systemen bzw. Chiplabor-Systemen oder Chiplabors bzw. mikrofluidischen Systemen abgegeben.

Mikrofluidische Lab-on-a-Chip-Systeme bestehen beispielsweise häufig aus Mehrschichtaufbauten mit integrierten mikrofluidischen Kanälen, Kavitäten, Ventilen und Filtereinrichtungen. Hierdurch wird es beispielsweise ermöglicht, konventionelle diagnostische Assays zu integrieren und automatisiert ablaufen zu lassen, wodurch sich Zeitvorteile und Kostenvorteile ergeben können. Zu zentralen Schritten bei einer Aufbereitung einer Patientenprobe, wie zum Beispiel Blut, Urin oder Sputum, zum Nachweis von Zellen, beispielsweise Bakterien oder zirkulierenden Tumorzellen, gehört eine Isolation der Zellen aus der Probe, um störende Restbestandteile zu entfernen, und ein anschließendes Lysieren der Zellen, d. h. ein Öffnen einer Zellmembran, um darin befindliche Bestandteile, wie z. B. DNA oder Proteine, freizusetzen, um deren eindeutige Identifizierung zu ermöglichen.

Zur Isolation der Zellen sind verschiedene Prinzipien üblich. Bei einer mechanischen Filtrierung durch Größenausschluss werden Filtermaterialien eingesetzt, deren Porendurchmesser groß genug ist, um andere Bestandteile der Probe durchzuleiten, jedoch klein genug, um die Zielzellen zurückzuhalten. Bei einer Zentrifugation wird, makroskopisch und mikrofluidisch betrachtet, eine unterschiedliche Dichte von Zielzellen zur Umgebung ausgenutzt, um durch Beschleunigungskräfte Zellen zu separieren. Bei einer hydrodynamischen Trennung können, mikrofluidisch betrachtet, hydrodynamische Kräfte, welche insbesondere in mikrofluidischen Kanälen auftreten, genutzt werden, um Zellen zu isolieren. Bei einer Verwendung akustischer Filter können, mikrofluidisch betrachtet, stehende Wellen eingesetzt werden, um Partikel innerhalb von Wellenbäuchen oder Wellenknoten zu sammeln und mit Hilfe eines überlagerten Flusses in verschiedene Kanäle zu leiten.

Für eine Lyse der Zellen sind verschiedene Prinzipien üblich. Bei einer thermischen Lyse zerreißt, makroskopisch und mikrofluidisch betrachtet, ab einer gewissen Temperatur, beispielsweise im Bereich von 100 Grad Celsius, die Zellmembran. Bei einer enzymatischen Lyse können makroskopisch und mikrofluidisch betrachtet Substanzen eingesetzt werden, welche die Zellmembran mit Hilfe einer chemischen Reaktion zersetzen. Bei einer Lyse mittels Ultraschall kann, mikrofluidisch betrachtet und unter Verwendung eines externen Ultraschallgebers, die Probe bzw. Flüssigkeit mit den intakten Zielzellen in einer Kavität innerhalb eines mikrofluidischen Systems vorgehalten werden, an welches von außen eine Ultraschallsonotrode in Kontakt gebracht wird. Über eine Wand der Kavität wird dann mit Hilfe der Sonotrode Ultraschallenergie in die Probe bzw. Flüssigkeit eingebracht. Damit einhergehende Druckwellen führen dazu bzw. der Effekt der Kavitation führt dazu, dass die Zielzellen lysiert werden. Bei einer Lyse mittels Ultraschall kann, mikrofluidisch betrachtet und unter Verwendung eines integrierten Ultraschallgebers, ein ähnliches Grundprinzip wie vorstehend zum Einsatz kommen, jedoch mit einem in das mikrofluidische System integrierten Ultraschallgeber, beispielsweise in Form einer Schicht aus Blei-Zirkonat-Titanat (PZT).

Fig. 1 zeigt eine schematische Schnittdarstellung einer Bearbeitungsvorrichtung 100 bzw. Vorrichtung zum Bearbeiten gemäß einem Ausführungsbeispiel der vorliegenden Erfindung. Die Bearbeitungsvorrichtung 100 ist ausgebildet, um eine Zielzellen enthaltende Probe biologischen Materials zu bearbeiten. Die Probe ist hierbei eine flüssige Probe, welche die Zielzellen und beispielsweise Begleitzellen und/oder Begleitpartikel aufweist. Dabei ist die Bearbeitungsvorrichtung 100 beispielsweise Teil eines mikrofluidischen Systems, insbesondere zur Verwendung für medizinische Diagnostik. Die Bearbeitungsvorrichtung 100 ist gemäß dem in Fig. 1 dargestellten Ausführungsbeispiel der vorliegenden Erfindung in Gestalt eines Schichtstapels ausgeführt.

Die Bearbeitungsvorrichtung 100 weist gemäß dem in Fig. 1 dargestellten Ausführungsbeispiel der vorliegenden Erfindung ein erstes Substrat 101 und ein zweites Substrat 102 auf. In dem ersten Substrat 101 ist ein erster mikrofluidischer Kanal 103 ausgeformt. Der erste mikrofluidische Kanal 103 ist beispielsweise ein Zufuhrkanal für die Probe. In dem zweiten Substrat 102 ist ein zweiter mikrofluidischer Kanal 104 ausgeformt. Der zweite mikrofluidische Kanal 104 ist beispielsweise ein Abfuhrkanal für die Probe. Ferner weist das erste Substrat 101 einen ersten Durchbruch 105 bzw. eine erste Kavität auf. Der erste Durchbruch 105 ist als eine Durchgangsöffnung in dem ersten Substrat 101 ausgeformt. Dabei mündet der erste mikrofluidische Kanal 103 in den ersten Durchbruch 105. Das zweite Substrat 102 weist einen zweiten Durchbruch 106 bzw. eine zweite Kavität auf. Der zweite Durchbruch 106 ist als eine Durchgangsöffnung in dem zweiten Substrat 102 ausgeformt. Hierbei mündet der zweite mikrofluidische Kanal 104 in den zweiten Durchbruch 106. Die mikrofluidischen Kanäle 103 und 104 sowie die Durchbrüche 105 und 106 sind ausgebildet, um die Probe biologischen Materials zu führen.

Auch weist die Bearbeitungsvorrichtung 100 eine Membran 107 mit einer Mehrzahl von Poren 108 auf. Aus Platzgründen ist in der Darstellung lediglich eine Teilmenge der Poren 108 mit Bezugszeichen versehen. Die Mehrzahl von Poren 108 ist ausgebildet, um die Zielzellen aus der Probe zu filtern. Die Membran 107 ist als eine Filtermembran aus einem piezoelektrischen Material ausgeformt. Dabei ist die Membran 107 bzw. Filtermembran ausgebildet, um durch ein an die Membran 107 angelegtes oder anlegbares elektrisches Signal mechanisch verformt zu werden. Insbesondere ist die Membran 107 ausgebildet, um durch das elektrische Signal in mechanische Schwingungen versetzt zu werden. Die mechanische Verformung ist bzw. die mechanischen Schwingungen der Membran 107 sind geeignet, um eine Lyse der Zielzellen zu bewirken.

Das erste Substrat 101, das zweite Substrat 102 und die Membran 107 sind hierbei gemäß dem in Fig. 1 gezeigten Ausführungsbeispiel der vorliegenden Erfindung aufeinander gestapelt angeordnet und miteinander verbunden. Dabei ist die Membran 107 zwischen dem ersten Substrat 101 und im zweiten Substrat 102 angeordnet. Genau gesagt sind hierbei der erste Durchbruch 105 des ersten Substrats 101 und der zweite Durchbruch 106 des zweiten Substrats 102 durch die Membran 107 getrennt. Anders ausgedrückt ist ein Teilabschnitt der Membran 107 zwischen dem ersten Durchbruch 105 und dem zweiten Durchbruch 106 angeordnet. Hierbei grenzt eine erste Hauptoberfläche der Membran 107 an den ersten Durchbruch 105 an, wobei eine zweite Hauptoberfläche der Membran 107 an den zweiten Durchbruch 106 angrenzt. Auch ist hierbei ein Randabschnitt der Membran 107 zwischen einem Korpus des ersten Substrats 101 und einem Korpus des zweiten Substrats 102 angeordnet.

Gemäß dem in Fig. 1 dargestellten Ausführungsbeispiel der vorliegenden Erfindung ist in dem ersten Substrat 101 eine Aussparung bzw. ein Aufnahmeabschnitt 109 zum Aufnehmen der Membran 107, insbesondere des Randabschnittes der Membran 107 ausgeformt. Somit ist der Randabschnitt der Membran 107 in dem Aufnahmeabschnitt 109 angeordnet. Der Aufnahmeabschnitt 109 ist durch eine Stufe in einer dem zweiten Substrat 102 zugewandten Hauptoberfläche des ersten Substrats 101 ausgeformt. Dabei ist der Aufnahmeabschnitt 109 den ersten Durchbruch 105 herum ausgeformt.

Gemäß einem anderen Ausführungsbeispiel kann zusätzlich oder alternativ das zweite Substrat 102 einen weiteren Aufnahmeabschnitt aufweisen.

Zum elektrischen Kontaktieren der Membran 107 weist die Bearbeitungsvorrichtung 100 ferner Leiterbahnen 110 und 111 bzw. elektrische Leiter auf. Die Leiterbahnen 110 und 111 sind elektrisch leitfähig mit der Membran 107 verbunden. Dabei sind die Leiterbahnen 110 und 111 zwischen dem ersten Substrat 101 und dem zweiten Substrat 102 angeordnet. Gemäß dem in Fig. 1 gezeigten Ausführungsbeispiel der vorliegenden Erfindung sind die Leiterbahnen 110 und 111 an dem zweiten Substrat 102 aufgebracht und/oder angeordnet. Somit ist die Membran 107 auf Seiten einer dem zweiten Durchbruch 106 zugewandten Hauptoberfläche der Membran 107 durch die Leiterbahnen 110 und 111 elektrisch kontaktiert.

Zusätzlich weist die Bearbeitungsvorrichtung 100 gemäß dem in Fig. 1 dargestellten Ausführungsbeispiel der vorliegenden Erfindung eine erste Deckschicht 112 und eine zweite Deckschicht 113 auf. Die erste Deckschicht 112 ist an einer von dem zweiten Substrat 102 abgewandten Hauptoberfläche des ersten Substrats 101 aufgebracht und/oder angeordnet. Die zweite Deckschicht 113 ist an einer von dem ersten Substrat 101 abgewandten Hauptoberfläche des zweiten Substrats 102 aufgebracht und/oder angeordnet. Der erste mikrofluidische Kanal 103 ist hierbei durch die erste Deckschicht 112 abgedeckt. Der zweite mikrofluidische Kanal 104 ist dabei durch die zweite Deckschicht 113 abgedeckt.

Anders ausgedrückt zeigt Fig. 1 einen Querschnitt durch eine Bearbeitungsvorrichtung 100, welche die beiden Substrate 101 und 102 mit jeweils einem mikrofluidischen Kanal 103 und 104 und jeweils einem Durchbruch 105 und 106 aufweist. Dazwischen befindet sich die Filtermembran 107 mit piezoelektrischen Eigenschaften, welche von den Poren 108 durchsetzt ist, welche eine Filtration durch Größenausschluss ermöglichen. Zur Aufnahme der Filtermembran 107 ist beispielhaft in dem ersten Substrat 101 die Aussparung 109 ausgeformt. Die elektrische Kontaktierung der Filtermembran 107 erfolgt über die zwei Leiterbahnen 110 und 111, welche beispielhaft auf dem zweiten Substrat 102 aufgebracht sind. Die mikrofluidischen Kanäle 103 und 104 sind durch die Deckschichten 112 und 113 gedeckelt, um ein abgeschlossenes System zu erhalten. Für eine Abdichtung der Filtermembran 107 können beispielsweise geeignete Klebstoffe verwenden werden.

Fig. 2 zeigt eine schematische Schnittdarstellung einer Bearbeitungsvorrichtung 100 gemäß einem Ausführungsbeispiel der vorliegenden Erfindung. Die in Fig. 2 gezeigte Bearbeitungsvorrichtung 100 entspricht hierbei der Bearbeitungsvorrichtung aus Fig. 1 mit Ausnahme dessen, dass das erste Substrat 101 ohne Aufnahmeabschnitt ausgeformt ist und dass die Bearbeitungsvorrichtung 100 in Fig. 2 ferner eine Trägerschicht 214 in Gestalt einer elastischen Membran aufweist. Die Trägerschicht 214 ist zwischen dem ersten Substrat 101 und dem zweiten Substrat 102 angeordnet.

Anders ausgedrückt handelt es sich bei der Trägerschicht 214 um eine elastische Membran, welche zwischen den Substraten 101 und 102 eingefügt ist und im Bereich der Durchbrüche 105 und 106 eine Durchgangsöffnung aufweist bzw. geöffnet ist. Somit kann auf einen Aussparungsabschnitt in mindestens einem der Substrate 101 und 102 verzichtet werden, da eine Elastizität der Trägerschicht 214 so gewählt sein kann, dass in einem zusammengefügten Zustand der Bearbeitungsvorrichtung 100 eine Höhendifferenz zwischen der Trägerschicht 214 und der Membran 107 kompensiert werden kann. Hierdurch kann auch auf einen Einsatz von Klebstoffen im Randabschnitt der Membran 107 verzichtet werden.

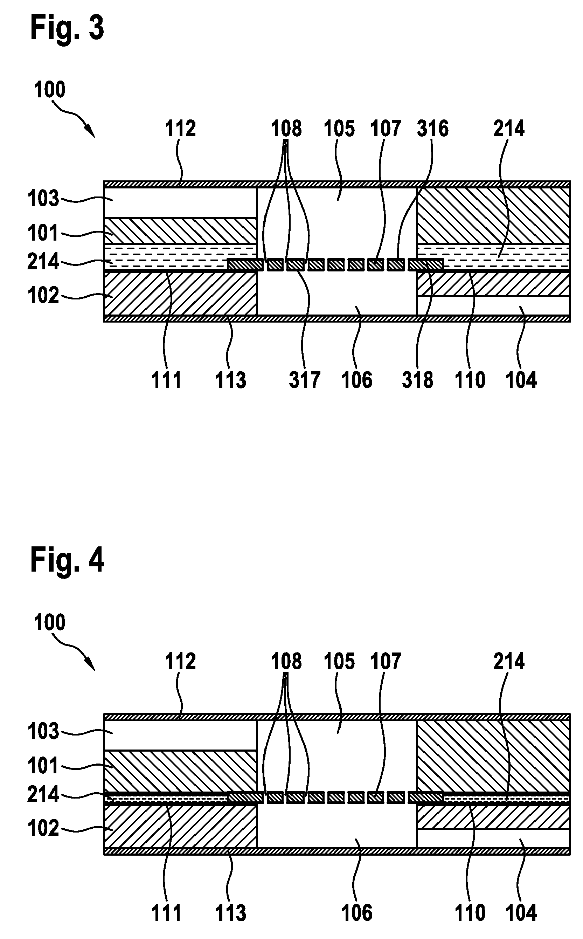

Fig. 3 zeigt eine schematische Schnittdarstellung einer Bearbeitungsvorrichtung 100 gemäß einem Ausführungsbeispiel der vorliegenden Erfindung. Die in Fig. 3 gezeigte Bearbeitungsvorrichtung 100 entspricht hierbei der Bearbeitungsvorrichtung aus Fig. 2 mit Ausnahme dessen, dass in Fig. 3 die Membran 107 an einer ersten Hauptoberfläche mit einer ersten elektrisch leitfähigen Schicht 316 und an einer zweiten Hauptoberfläche mit einer zweiten elektrisch leitfähigen Schicht 317 beschichtet ist und dass die Membran 107 einen Durchkontakt 318 zwischen der ersten Hauptoberfläche und der zweiten Hauptoberfläche der Membran 107 aufweist.

Die erste elektrisch leitfähige Schicht 316 ist auf Seiten des ersten Durchbruchs 105 bzw. des ersten Substrats 101 auf die Membran 107 aufgebracht. Die zweite elektrisch leitfähige Schicht 317 ist auf Seiten des zweiten Durchbruchs 106 bzw. des zweiten Substrats 102 auf die Membran 107 aufgebracht. Die erste elektrisch leitfähige Schicht 316 und die zweite elektrisch leitfähige Schicht 317 sind mittels des Durchkontakts 318 miteinander verbunden. Der Durchkontakt 318 dient dabei dazu, die obere Schicht über die Leiterbahn 110 kontaktieren zu können, jedoch nicht einer elektrisch leitfähigen Verbinnung der beiden Schichten .Dabei ist beispielhaft eine erste Leiterbahn 110 der Leiterbahnen 110 und 111 an dem Durchkontakt 318 elektrisch angeschlossen. Somit ist die erste elektrisch leitfähige Schicht 316 durch die erste Leiterbahn 110 über den Durchkontakt 318 kontaktiert, wobei die zweite elektrisch leitfähige Schicht 317 durch eine zweite Leiterbahn 111 der Leiterbahnen 110 und 111 kontaktiert ist.

Die Membran 107 ist somit beidseitig mit leitfähigem Material beschichtet. Die elektrisch leitfähigen Schichten 316 und 317 sind dabei über den Poren 108 geöffnet und der Art elektrisch kontaktiert, dass die Leiterbahnen 110 und 111 lediglich jeweils zu einer der beiden elektrisch leitfähigen Schichten 316 und 317 in Kontakt sind. Der Durchkontakt 318 ist hierbei ein mit leitfähigem Material gefüllter Durchbruch durch die Membran 107 ausgeführt.

Fig. 4 zeigt eine schematische Schnittdarstellung einer Bearbeitungsvorrichtung 100 gemäß einem Ausführungsbeispiel der vorliegenden Erfindung. Die in Fig. 4 gezeigte Bearbeitungsvorrichtung 100 entspricht hierbei der Bearbeitungsvorrichtung aus Fig. 3 mit Ausnahme dessen, dass in Fig. 4 die Membran 107 ohne Durchkontakt ausgeführt ist, wobei anders ausgedrückt auf den Einsatz eines mit leitfähigem Material gefüllten Durchbruchs innerhalb der Membran 107 verzichtet wird, und dass die erste Leiterbahn 110 auf das erste Substrat 101 aufgebracht ist, wobei hierdurch können Kosten für die Membran 107 reduziert werden.

Somit ist die erste Leiterbahn 110 auf Seiten einer ersten Hauptoberfläche der Membran 107 angeordnet. Die erste Leiterbahn 110 ist somit angeordnet, um die Membran 107 an einer dem ersten Durchbruch 105 bzw. dem ersten Substrat 101 zugewandten Hauptoberfläche der Membran 107 elektrisch zu kontaktieren.

Die zweite Leiterbahn 111 ist auf Seiten einer zweiten Hauptoberfläche der Membran 107 angeordnet. Die zweite Leiterbahn 111 ist somit angeordnet, um die Membran 107 an einer dem zweiten Durchbruch 106 bzw. dem zweiten Substrat 102 zugewandten Hauptoberfläche der Membran 107 elektrisch zu kontaktieren. Der Aussparungsabschnitt der Trägerschicht 214 ist hierbei als eine Durchgangsöffnung ausgeformt.

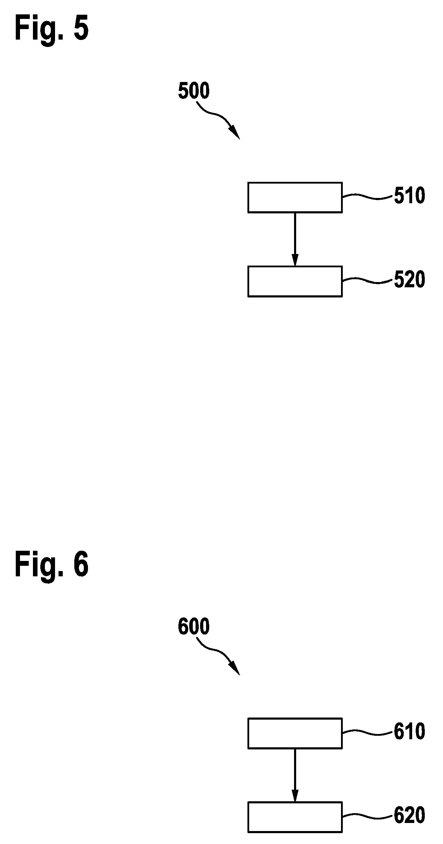

Fig. 5 zeigt ein Ablaufdiagramm eines Verfahrens 500 zum Herstellen gemäß einem Ausführungsbeispiel der vorliegenden Erfindung. Das Verfahren 500 zum Herstellen ist ausführbar, um eine Bearbeitungsvorrichtung bzw. Vorrichtung zum Bearbeiten einer Zielzellen enthaltenden Probe biologischen Materials herzustellen. Durch Ausführen des Verfahrens 500 zum Herstellen ist eine Bearbeitungsvorrichtung aus einer der Figuren 1 bis 4 oder eine ähnliche Bearbeitungsvorrichtung herstellbar.

Das Verfahren 500 weist einen Schritt 510 des Ausformens einer Membran aus einem piezoelektrischen Material auf. Dabei ist die im Schritt 510 des Ausformens ausgeformte Membran ausgebildet, um durch ein elektrisches Signal mechanisch verformbar zu sein, um eine Lyse der Zielzellen zu bewirken.

Auch weist das Verfahren 500 einen Schritt 520 des Ausbildens von Poren in der ausgeformten Membran auf. Dabei werden die Poren im Schritt 520 des Ausbildens so ausgebildet, dass sie zum Filtern der Zielzellen aus der Probe geeignet sind. Die Poren und gegebenenfalls andere Strukturen innerhalb der Membran können mikromechanisch eingebracht werden, insbesondere durch nasschemisches Ätzen oder dergleichen.

Gemäß einem Ausführungsbeispiel können bei dem Verfahren 500 auch Leiterbahnen beispielsweise unter Verwendung von Siebdruck, Inkjet-Printing, Moulded Interconnect Devices Technologie (MID-Technologie), insbesondere durch Sputtern oder Aufdampfen, aufgebracht werden. Ferner können bei dem Verfahren 500 zudem Schichten zum Ausbilden eines Schichtstapels der Bearbeitungsvorrichtung beispielsweise unter Verwendung von Klebstoffen, Laserdurchstrahlschweißen oder thermischem Bonden miteinander gefügt werden.

Fig. 6 zeigt ein Ablaufdiagramm eines Verfahrens 600 zum Bearbeiten gemäß einem Ausführungsbeispiel der vorliegenden Erfindung. Das Verfahren 600 zum Bearbeiten ist ausführbar, um eine Zielzellen enthaltende Probe biologischen Materials zu bearbeiten. Dabei ist das Verfahren 600 zum Bearbeiten unter Verwendung von bzw. in Verbindung mit einer Bearbeitungsvorrichtung wie der Bearbeitungsvorrichtung aus einer der Figuren 1 bis 4 ausführbar. Anders ausgedrückt ist das Verfahren 600 zum Bearbeiten ausführbar, um eine Filtration und eine Lyse einer Zielzellen enthaltenden Probe biologischen Materials durchzuführen.

Das Verfahren 600 zum Bearbeiten weist einen Schritt 610 des Filterns der Zielzellen aus der Probe unter Verwendung der Membran mit den Poren auf. Dazu kann bei dem Verfahren 600 zum Bearbeiten die Probe, insbesondere flüssige Probe, welche die Zielzellen enthält und deren Hintergrund entfernt werden soll, über den ersten mikrofluidischen Kanal in die Bearbeitungsvorrichtung eingebracht werden. Der Schritt 610 des Filterns kann beispielsweise druckgetrieben erfolgen, z. B. durch in ein mikrofluidisches System integrierte Pumpen oder externe Pumpen. Im Schritt 610 des Filterns werden die Zielzellen auf der Membran der Bearbeitungsvorrichtung zurückgehalten und wird ein Filterdurchgangsmaterial der Probe über den zweiten mikrofluidischen Kanal der Bearbeitungsvorrichtung abtransportiert.

In einem bezüglich des Schrittes 610 des Filterns nachfolgend ausführbaren Schritt 620 des Anlegens wird bei dem Verfahren 600 zum Bearbeiten ein elektrisches Signal an die aus dem piezoelektrischen Material ausgeformte Membran angelegt, um die Membran mechanisch zu verformen, um so eine Lyse der Zielzellen zu bewirken. Für den Schritt 620 kann ein Lysepuffer bzw. eine Flüssigkeit, welche eine geeignete chemische Umgebung für die Lyse einstellen kann, zu den herausgefilterten Zielzellen zugegeben werden. Im Schritt 620 des Anlegens kann eine elektrische Spannung an die Leiterbahnen der Bearbeitungsvorrichtung angelegt werden. Hierdurch kommt es aufgrund des piezoelektrischen Effekts zu einer mechanischen Deformation der Membran, welche es ermöglicht, mit Hilfe entstehender Druckwellen bzw. Kavitation die Zielzellen zu lysieren.

Unter Bezugnahme auf die Figuren 1 bis 6 wird nachfolgend auf beispielhafte Prinzipien und Grundlagen von Ausführungsbeispiel der vorliegenden Erfindung zusammenfassend und mit anderen Worten eingegangen.

Die Bearbeitungsvorrichtung 100 repräsentiert einen Schichtaufbau bestehend aus zwei Substraten 101 und 102 mit jeweils einem Kanal 103 und 104 und jeweils einem Durchloch bzw. Durchbruch 105 und 106, sowie einer zwischen den Substrat 101 und 102 angeordneten piezoelektrischen Schicht bzw. Membran 107, die elektrisch kontaktiert ist. Die piezoelektrische Membran 107 ist zusätzlich durchsetzt von Poren 108, deren Durchmesser eine Filtrierung von Zellen der Probe durch Größenausschluss ermöglicht. Auf diese Weise kann zunächst eine Probe druckgetrieben durch den durch die Membran 107 repräsentierten Filter gespült werden, woraufhin die Zielzellen auf der piezoelektrischen Filterschicht bzw. Membran 107 zurückgehalten werden. Nachfolgend kann durch Einbringung eines geeigneten elektrischen Signals die piezoelektrische Filterschicht bzw. Membran 107 mechanisch bewegt bzw. verformt werden, um die Zielzellen durch die so bewirkten Druckwellen bzw. die so bewirkte Kavitation zu lysieren. Hierbei wird mit der Membran 107 ein einzelnes Bauteil bereitgestellt oder verwendet, um die Funktionen der Filtrierung und Lyse teilemäßig kombiniert durchzuführen.

Materialbeispiele für Teile der Bearbeitungsvorrichtung 100 umfassen für die Substrate 101 und 102 beispielsweise ein Polymermaterial, z. B. Thermoplast (z. B. PC, PP, PE, PMMA, COP, COC) oder Duroplast, Silizium oder Glas, für die Deckschichten 112 und 113 beispielsweise Silizium, Glas, Polymerfolien, insbesondere Klebefolien, oder dergleichen, für die Leiterbahnen 110 und 111 sowie für die elektrisch leitfähigen Schichten 316 und 317 sowie den Durchkontakt 318 ein leitfähiges Material, insbesondere ein Metall oder Metalle, z. B. Kupfer, Aluminium, Titan oder dergleichen, und für die Membran 107 schließlich piezoelektrische Materialien, insbesondere PZT oder dergleichen.

Beispielhafte Abmessungen für Teile der Bearbeitungsvorrichtung 100 umfassen für eine Dicke der Substrate 101 und 102 beispielsweise 0,1 bis 10 Millimeter, für eine Dicke der Membran 107 beispielsweise 50 Mikrometer bis 1 Millimeter, für eine Fläche der Membran 107 beispielsweise 500 Quadratmikrometer bis 100 Quadratmillimeter, für einen Durchmesser der Poren 108 beispielsweise 50 Nanometer bis 10 Mikrometer und für eine Dicke der Trägerschicht 214 beispielsweise 100 Mikrometer bis 2 Millimeter.

Beispielhafte elektrische Parameter für ein Ansteuern der piezoelektrischen Filtermembran bzw. Membran 107 in dem Schritt 620 des Anlegens bei dem Verfahren 600 zum Bearbeiten umfassen eine elektrische Wechselspannung mit einer Frequenz von beispielsweise 1 Kilohertz bis 10 Megahertz und einer Spannung von beispielsweise 100 Volt bis 5000 Volt.

Die beschriebenen und in den Figuren gezeigten Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können die hier vorgestellten Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Vorrichtung (100) zum Bearbeiten einer Zielzellen enthaltenden Probe biologischen Materials, wobei die Vorrichtung (100) folgende Merkmale aufweist:
eine Membran (107) mit Poren (108), wobei die Poren (108) ausgebildet sind, um die Zielzellen aus der Probe zu filtern, wobei die Membran (107) aus einem piezoelektrischen Material ausgeformt ist, wobei die Membran (107) ausgebildet ist, um durch ein elektrisches Signal mechanisch verformt zu werden, um eine Lyse der Zielzellen zu bewirken.

2. Vorrichtung (100) gemäß Anspruch 1, **dadurch gekennzeichnet, dass** die Membran (107) zumindest an einer ersten Hauptoberfläche mit einer ersten elektrisch leitfähigen Schicht (316) und an einer zweiten Hauptoberfläche mit einer zweiten elektrisch leitfähigen Schicht (317) beschichtet ist.

3. Vorrichtung (100) gemäß einem der vorangegangenen Ansprüche, **gekennzeichnet durch** elektrische Leiter (110, 111) zum elektrischen Kontaktieren der Membran (107), wobei ein erster elektrischer Leiter (110) auf Seiten einer ersten Hauptoberfläche der Membran (107) angeordnet ist und/oder ein zweiter elektrischer Leiter (111) auf Seiten einer zweiten Hauptoberfläche der Membran (107) angeordnet ist.

4. Vorrichtung (100) gemäß einem der Ansprüche 1 bis 2, **gekennzeichnet durch** elektrische Leiter (110, 111) zum elektrischen Kontaktieren der Membran (107), wobei die elektrischen Leiter (110, 111) auf Seiten einer ersten Hauptoberfläche der Membran (107) angeordnet sind, wobei die Membran (107) einen Durchkontakt (318) zwischen der ersten Hauptoberfläche und einer zweiten Hauptoberfläche der Membran (107) aufweist, insbesondere wobei einer der elektrischen Leiter (110) an dem Durchkontakt (318) angeschlossen ist.

5. Vorrichtung (100) gemäß einem der vorangegangenen Ansprüche, **gekennzeichnet durch** eine Trägerschicht (214) mit einem Aussparungsabschnitt zum Aufnehmen der Membran (107), wobei die Membran (107) in dem Aussparungsabschnitt der Trägerschicht (214) angeordnet ist.

6. Vorrichtung (100) gemäß einem der vorangegangenen Ansprüche, **gekennzeichnet durch** ein erstes Substrat (101) mit einer ersten Kavität (103, 105), ein zweites Substrat (102) mit einer zweiten Kavität (104, 106) und elektrische Leiter (110, 111) zum elektrischen Kontaktieren der Membran (107), wobei die Substrate (101, 102) zusammengefügt sind, wobei die Membran (107) und die elektrischen Leiter (110, 111) zwischen den Substraten (101, 102) angeordnet sind, wobei mittels der Membran (107) die Kavitäten (103, 104, 105, 106) voneinander abgetrennt sind.

7. Vorrichtung (100) gemäß Anspruch 6, **dadurch gekennzeichnet, dass** zumindest eines der Substrate (101, 102) einen Aufnahmeabschnitt (109) zum Aufnehmen der Membran (107) aufweist, wobei die Membran (107) in dem Aufnahmeabschnitt (109) angeordnet ist.

8. Verfahren (500) zum Herstellen einer Vorrichtung (100) zum Bearbeiten einer Zielzellen enthaltenden Probe biologischen Materials, wobei das Verfahren (500) folgende Schritte aufweist:
Ausformen (510) einer Membran (107) aus einem piezoelektrischen Material, wobei die Membran (107) durch ein elektrisches Signal mechanisch verformbar ist, um eine Lyse der Zielzellen zu bewirken; und
Ausbilden (520) von Poren (108), die zum Filtern der Zielzellen aus der Probe geeignet sind, in der Membran (107).

9. Verfahren (600) zum Bearbeiten einer Zielzellen enthaltenden Probe biologischen Materials, wobei das Verfahren (600) in Verbindung mit einer Vorrichtung (100) gemäß einem der vorangegangenen Ansprüche ausführbar ist, wobei das Verfahren (600) folgende Schritte aufweist:
Filtern (610) der Zielzellen aus der Probe unter Verwendung der Membran (107) mit den Poren (108); und
Anlegen (620) eines elektrischen Signals an die aus dem piezoelektrischen Material ausgeformte Membran (107), um die Membran (107) mechanisch zu verformen, um eine Lyse der Zielzellen zu bewirken.

10. Steuergerät, das ausgebildet ist, um alle Schritte eines Verfahrens (500; 600) gemäß einem der vorangegangenen Ansprüche durchzuführen, umzusetzen und/oder anzusteuern.

11. Computerprogramm, das dazu eingerichtet ist, alle Schritte eines Verfahrens (500; 600) gemäß einem der vorangegangenen Ansprüche durchzuführen, umzusetzen und/oder anzusteuern.

12. Maschinenlesbares Speichermedium mit einem darauf gespeicherten Computerprogramm nach Anspruch 11.
